# EUROPEAN PATENT APPLICATION

(11) **EP 3 333 255 A1**
(43) Date of publication of application: **13.06.2018**
(21) Application number: 16766331.9
(22) Date of filing: 08.08.2016
(51) Int. Cl.: C12N 5/0783, C12N 5/0784, A61K 39/00, A61K 35/74, A61K 35/13

(54) **TRANSINFECTED LYMPHOCYTES FOR ANTI-TUMOR THERAPY**

(30) Priority: 07.08.2015 ES 201531177
(71) Applicant: Consejo Superior De Investigaciones Científicas, 28006 Madrid (ES); Universidad Autónoma de Madrid, 28049 Madrid (ES); Fundación Para La Investigación Biomédica Del Hospital De La Princesa, 28006 Madrid (ES)
(72) Inventor: VEIGA CHACÓN, Esteban, Cantoblanco - 28049 Madrid (ES); CRUZ ADALIA, Aranzazu, Cantoblanco - 28049 Madrid (ES); RAMÍREZ SANTIAGO, Guillermo, Cantoblanco - 28049 Madrid (ES); ALARCÓN SÁNCHEZ, Balbino, 28049 Madrid (ES); SÁNCHEZ MADRID, Francisco, 28006 Madrid (ES)
(74) Representative: Pons
(86) International application number: PCT/ES2016/070597
(87) International publication number: WO 2017/025657

(57) **Abstract**

The present invention relates to lymphocytes, preferably that have been transinfected from dendritic cells with a bacterium, preferably CD4⁺ T cells, preferably *Listeria monocytogenes,* wherein said bacterium comprises a tumor peptide. It also relates to the use of lymphocytes for therapy and/or treatment of solid tumors, preferably melanoma, the kit or device which comprises for this purpose. Furthermore, it also refers to the transinfection method thereof.

## Description

The present invention relates to lymphocytes, preferably CD4⁺ T cells that have been transinfected from dendritic cells with a bacterium, preferably *Listeria monocytogenes,* wherein said bacterium comprises a tumor antigen. The present invention could be framed in the field of medicine.

### STATE OF THE ART

Activation of CD8⁺T cells in response against cancer and infectious diseases is carried out mainly by dendritic cells (DCs), considered the most efficient and physiologically relevant antigen-presenting cells (APCs) (Joffre *et al.,* 2012). CD8⁺ T effector cells need to be activated by exogenous antigens exposed on the major histocompatibility complex I (MHC-I) at the surface of professional APCs. This process is known as cross-presentation (Joffre *et al.,* 2012). Until now it was thought that the cross-presentation was conducted exclusively by professional phagocytes of the innate immunity, mostly by DCs, considered the most efficient APCs. Antigen presentation occurs during formation of the immune synapse (IS), a structure formed by the intimate contact of a T cell (which will be activated) with an APC loaded with antigen recognizing this T cell (Saito and Batista, 2010). The formation of the IS induces activation of the T cell and is essential for an appropriate adaptive immune response.

In a recent study it was found that CD4⁺ T cells are able to capture bacteria, during the formation of the IS, which infect DCs in a process we called transinfection (Cruz-Adalia *et al.,* 2014). This transinfection through IS is various orders of magnitude more efficient than direct infection. In addition, it was found that transinfected T cells (tiT) kill engulfed bacteria more efficiently that professional phagocytes and also secrete large amounts of proinflammatory cytokines such as IL-6, interferon-y and TNF-α ; these properties confer protection in mice against infection by *Listeria monocytogenes.*

Beyond the classical forms of tumor treatments such as radiotherapy, chemotherapy, and biologic therapies, for example with antibodies (eg anti-PD-1 and CTLA-4) (Wolchok and Chan, 2014), there are emerging therapies involving adoptive transfer of leukocytes (Chen and Mellman, 2013).

Induction of systemic anti-tumor immunity requires the activation of CD8⁺ T cells specific for tumor associated antigens, that once activated can kill tumor cells. Results from clinical studies support the anti-tumor properties of DCs (Restifo *et al.,* 2012). DCs, which are considered the best antigen presenting cells, must capture, process and cross-present tumor antigens on their MHC-I to activate the CD8⁺ T cells that will finally eliminate malignant cells. Therefore, DCs have also been used as a vaccine against cancer (by adoptive transfer) because they are able to generate anti-tumor immune response *in vivo.* Currently, there are leukemia patients resistant to chemotherapy that have presented durable and complete clinical responses injecting cells by adoptive transfer (Kalos and June, 2013), showing that it is possible to achieve tumor immunotherapy sustainable in the long term.

Several mechanisms have been described that allow tumor cells to escape immune destruction. Although tumors express antigens, these are not removed by the host immune system. It could be because the antigens are not presented efficiently and therefore do not cause a sufficiently strong immune response or that a continuous selection in the cancer patient, so that tumor cells can evade immune recognition. In antitumor therapy becomes necessary therefore the appearance of a new tool to improve response against tumors activating CD8⁺ T cells against specific tumor antigens.

### DESCRIPTION OF THE INVENTION

In the present invention it is demonstrated the use of lymphocytes transinfected with bacteria (used as proof of concept *L. monocytogenes)* that express tumor antigens in tumor therapy. In an experimental model of aggressive melanoma (B-16 OVA), it is shown that vaccination of mice with CD4⁺ T cells transinfected with bacteria expressing exogenous antigens, also present in the tumor, confer protection against tumorigenesis. Furthermore it is shown that transinfected CD4⁺ lymphocytes activate CD8⁺ cytotoxic lymphocytes directly as APCs, ie by cross-presentation via MHC-I, and they are surprisingly much better presenters that any APCs known to date.

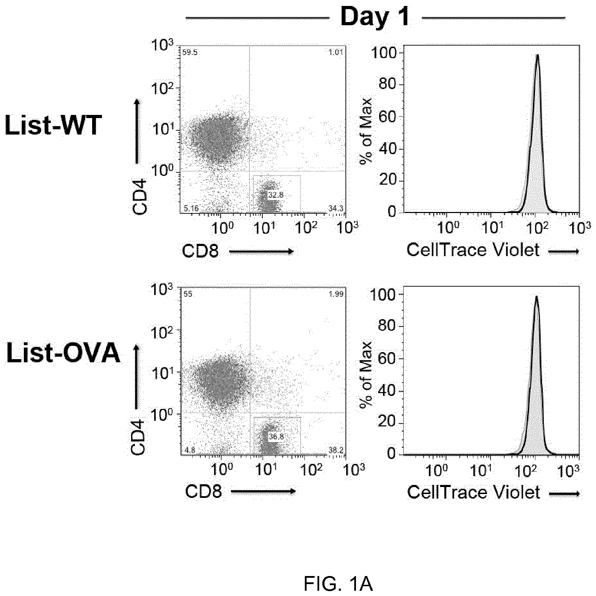

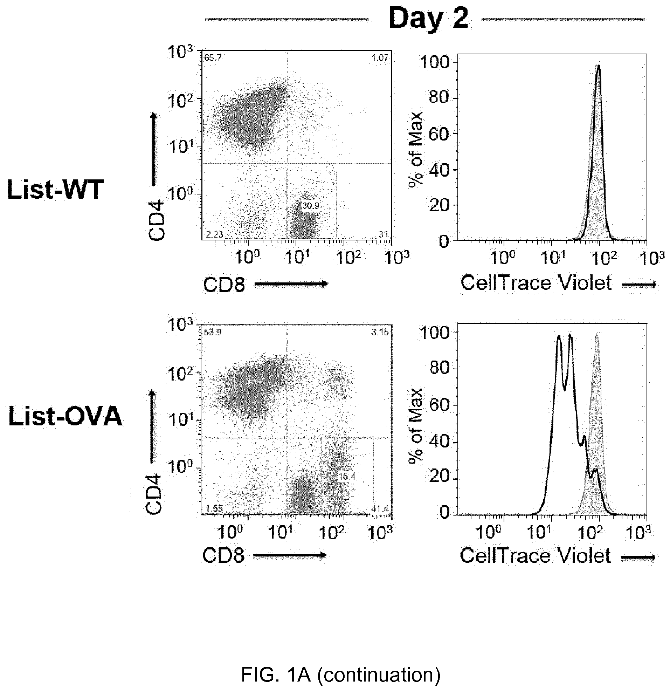

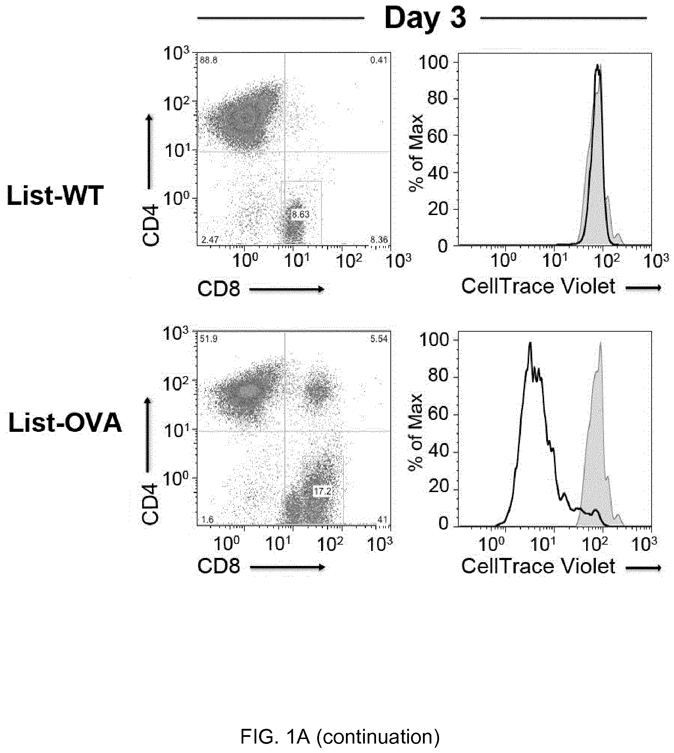

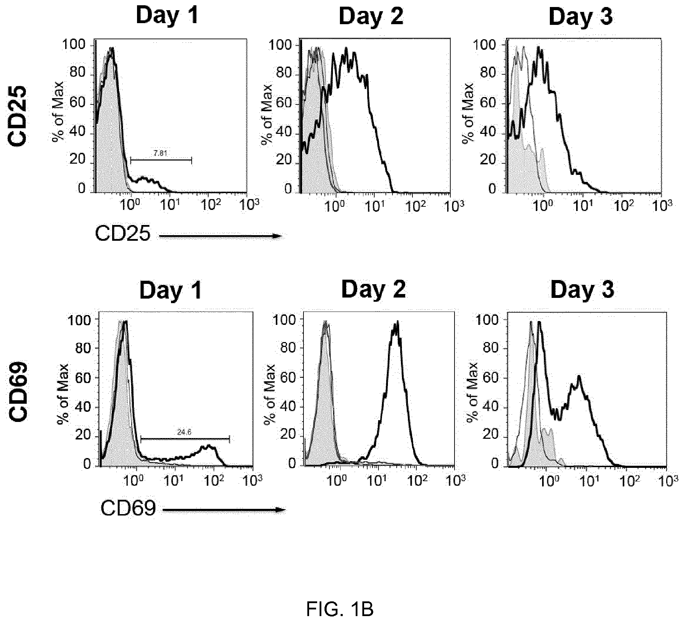

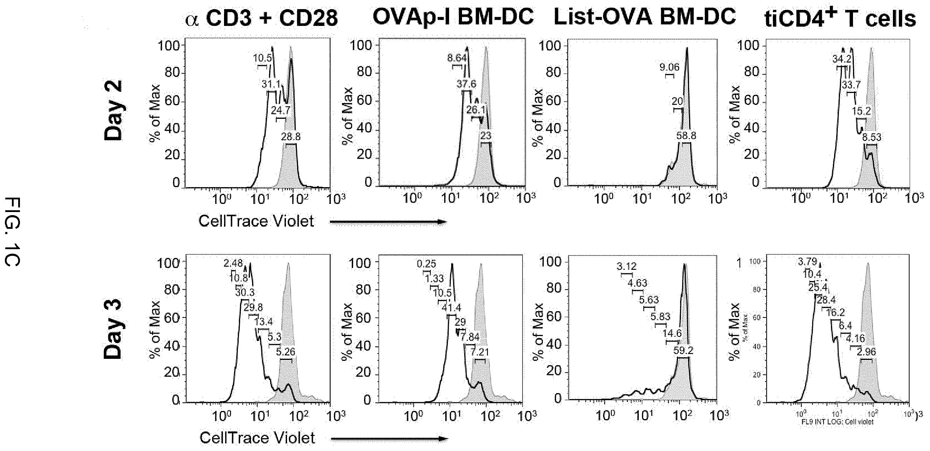

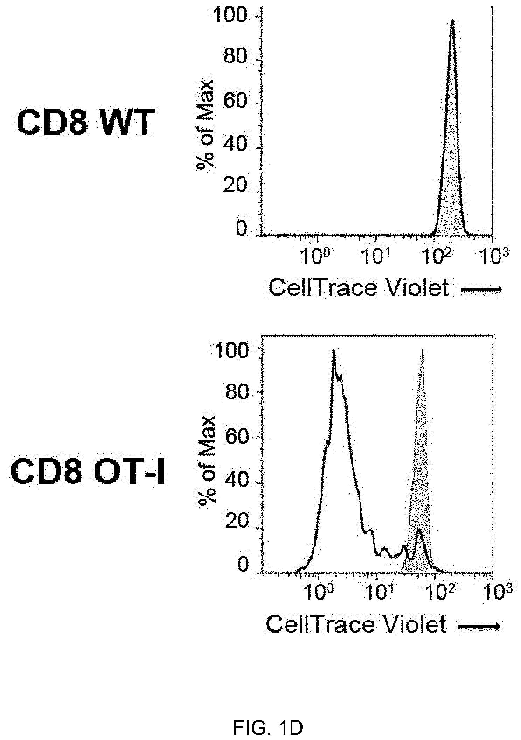

In the present invention it is demonstrated that tiT cells are capable of presenting antigens. It is demonstrated that CD4⁺ T transinfected with *Listeria-OVA (Listeria monocytogenes* expressing ovalbumin (Pope *et al.,* 2001)) are capable of activating strongly CD8⁺ *naive* cells from mice OT-I (transgenic mice where all T cell receptors (TCR) recognize a peptide of OVA (257-264; SIINFEKL) in the context of H-2K^{b} (Clarke *et al.,* 2000)) (Figure 1), and therefore *bonafide* APCs. It is also probed that tiT cells are able to internally process the bacteria captured, and antigen presentation is independent of the DC, in other words, that antigens do not come from the donor DCs but by cross-presentation by the tiT cells. These results show that conventional CD4⁺ lymphocytes, paradigm of adaptive immunity, are able to carry out roles previously thought unique to the cells of innate immunity.

In a first aspect, the present invention relates to a transinfected lymphocyte with a bacterium comprising a tumor antigen. Hereafter referred to as the "lymphocyte of the invention".

In a preferred embodiment of the first aspect of the invention the lymphocyte is a CD4⁺ T lymphocyte or a B lymphocyte

It is understood by CD4⁺ T cells those lymphocytes expressing T cell receptor (TCR), plus CD3 and coreceptor CD4. They are also called T *helper* lymphocytes.

It is understood by B cell those lymphocytes expressing B cell receptor; BCR. Other molecules used as B cell markers are CD19 and CD20.

Bacteria described in the present invention can be both Gram + and Gram -, and also bacteria may be pathogenic or nonpathogenic. In a more preferred embodiment the bacterium is selected from the list consisting of: *L. monocytogenes, Salmonella enterica,* bacteria of the *Mycobacterium tuberculosis* complex, *Staphylococcus aureus* and *Escherichia coli;* preferably is *L. monocytogenes.* Among the bacteria of the M. *tuberculosis* complex are included *M*. *tuberculosis, M. bovis* (BCG), *M*. *africanum, M. caprae, M. pinnipedii, M. canetti* and *M*. *microti.* In a particular embodiment is *L*. *monocytogenes.*

The term cancer in the present invention includes solid tumors and hematological tumors, such as leukemias, lymphomas, and myelodysplastic syndromes.

In a preferred embodiment, the lymphocyte of the first aspect of the invention is transinfected from a dendritic cell. In a more preferred embodiment the lymphocyte is from the same patient as the dendritic cell used for transinfection of said lymphocyte both cells are preferably originating from a patient with a tumor (tumor wherein said tumor comprises the antigenic peptide).

Transinfection means the process by which a lymphocyte (T or B) capture bacteria from APCs (preferably DC) previously infected.

APC (or antigen-presenting cell) is understood as those cells of the immune system whose function is to capture, process, and, as its name suggests, presenting antigens to the cells of the innate immune system through the histocompatibility complexes. In the present invention the APC is preferably a dendritic cell.

It is understood as APC a dendritic cell, whose origin can be both myeloid and lymphoid, specialized in presenting antigens. It expresses CD11c (in mice) and there are several subtypes, DC from thymus, conventional or plasmacytoid, and each has subtype specific markers. In the experiments underlying this invention DC are derived from bone marrow grown in the presence of GM-CSF (granulocyte and macrophages colony-stimulating factor). Markers of these cells were CD11c+, GR-, CD3-, CD19-, MHC-II +. Regarding humans, the preferred DC are derived from peripheral blood after treatment with GM-CSF and IL-4.

In another more preferred embodiment the bacterium comprises a tumor antigen that may be any antigen that is expressed in a specific manner in malignant cells of any tumor, as is highlighted in recent studies that propose how to identify tumor antigens (Schumacher and Schreiber, 2015; Yadav *et al.,* 2014); for example antigens identified in peptides from melanoma, lymphoma, chronic lymphocytic leukemia, myeloma, breast, ovary, uterus, cervix, testis, prostate, colon, colorectal, pancreatic , stomach and gastrointestinal tumors, gastric cancer, liver tumor, kidney (comprised clear-cell renal-cell carcinoma), bladder, mouth cancer, pharynx, larynx, esophagus, lung (small and non-small cell), thyroid, glioblastoma, glioma, sarcoma, encephalon, brain, neuroblastoma and marrow, head and neck blastoma, bone and connective tissue. The tumor can be at any stage and may also be metastatic. Antigens derived from these tumors are known by skilled persons (Schumacher and Schreiber, 2015; Yadav *et al.,* 2014). Preferably the tumor has neo-antigens (antigens resulting from a mutation in tumor cells and therefore absent in other cells of the body). They can also show fetal antigens, absent in cells of the adult organism. For example, antigenic proteins derived from melanoma include without limitation: tyrosinase, gp75, gp100, Melan A/MART-1, TRP-2, MAGE family proteins *(melanoma-associated antigen):* GAGE and BAGE, NY-ESO-1, mutations in CDk4 and, β-catenin, MUM-1, p15.

It is understood by tumor antigen, as that one specific of cancer, which can be of any size and is absent in healthy body cells.

When speaking of a specific antigen of a particular tumor, for example a "specific antigen of melanoma", it encompasses antigens of any size that are frequently associated with the tumor (eg melanoma).

In another more preferred embodiment of the first aspect of the invention the lymphocyte is heterologous or autologous.

In another more preferred embodiment of the first aspect of the invention the APC, preferably the dendritic cell, is autologous or heterologous and can be from the same or different individual than the transinfected lymphocyte.

In the present invention when referring to a lymphocyte we also refer to fragments of the lymphocyte and derivatives thereof, such as any modification that encourages contact between tiT and TIL (T lymphocytes infiltrating the tumor, and therefore with tropism against tumor antigens).

The lymphocyte of the invention is a mammal, preferably from a human of any race, age or sex.

In the present invention when referring to a lymphocyte we also refer to a population of lymphocytes.

A second aspect of the invention relates to a cell population comprising the lymphocyte of the first aspect of the invention.

In the present invention the term "cell population" refers to a mixture of cells including lymphocytes of the invention, preferably a mixture of blood cells.

A third aspect of the invention relates to a pharmaceutical composition comprising the lymphocyte of the first aspect of the invention or the cell population of the second aspect of the invention.

The pharmaceutical composition is preferably a vaccine.

The term "pharmaceutical composition" herein refers to any substance used for prevention, diagnosis, alleviation, treatment or cure of diseases in humans or animals. The pharmaceutical composition of the invention can be used either alone or in combination with other pharmaceutical compositions. The term pharmaceutical and drug composition are used in this invention interchangeably. In the context of the present invention the pharmaceutical composition or medicament is characterized by comprising the lymphocyte of the invention in a therapeutically effective amount, so that the lymphocyte exercises its function in the tissue/cell target.

In the present invention the term "therapeutically effective amount" refers to the amount of lymphocytes calculated to produce the desired effect and, in general, will be determined, in the case of a therapeutic composition, by the compounds characteristics, the route, manner and frequency of administration thereof, and other factors, including age, condition of the patient and the severity of the alteration or disorder.

The term "carrier", as the excipient, refers to a substance used in the pharmaceutical composition or medicament to dilute any component of the present invention included therein to a specific volume or weight. Vehicle function is to facilitate the incorporation of other elements, allowing a better dosage and administration or give consistency and form to the composition. When the presentation is liquid, the pharmacologically acceptable carrier is the diluent. Pharmaceutically acceptable carriers that may be used in the pharmaceutical composition of the present invention are known to those skilled in the art.

In another particular embodiment, said pharmaceutical composition is prepared in solid form or aqueous suspension, in a pharmaceutically acceptable diluent.

The therapeutic composition provided by this invention can be administered by any appropriate route of administration for which said composition is formulated in the suitable pharmaceutical form for the chosen administration route. In a particular embodiment, administration of the therapeutic composition provided by this invention is administered parenterally, for example, intra-arterially, intravenously, orally, intraperitoneally or subcutaneously, preferably intravenously.

The composition may be administered in one or several doses during the duration of treatment in order to optimize the therapeutic effect. Note that a single administration of the tiT has more effect than repeated injections of DC.

A fourth aspect of the invention relates to the use of lymphocyte of the first aspect of the invention or of the cell population of the second aspect of the invention for the manufacture of a medicament, preferably for the prevention or treatment of tumor and/or stimulation of the immune response against a tumor antigen.

Therefore another aspect of the invention relates to the lymphocyte of the first aspect of the invention or of the cell population of the second aspect of the invention for use as a medicament, preferably a medicament for the prevention or treatment of tumor and/or stimulation of the immune response against a tumor antigen.

The term "prevention", as understood in the present invention is to avoid or reduce the appearance of a tumor. The term "treatment" involves cure, mitigate, reduce or combat damage caused by a tumor, downsize, reduce its aggressiveness, reduce or avoid its dispersal, or to stabilize the status of individuals.

In the present invention the tumor to be treated or prevented is any tumor, for example from the following list: melanoma, lymphoma, chronic lymphocytic leukemia, myeloma, breast, ovary, uterus, cervix, testis, prostate, colon, colorectal, pancreatic , stomach and gastrointestinal tumors, gastric cancer, liver tumor, kidney (comprised clear-cell renal-cell carcinoma), bladder, mouth cancer, pharynx, larynx, esophagus, lung (small and non-small cell), thyroid, glioblastoma, glioma, sarcoma, encephalon, brain, neuroblastoma and marrow, head and neck blastoma, bone and connective tissue. Preferably is melanoma.

In the present invention stimulating the immune response against a tumor antigen comprises stimulating CD8⁺ lymphocytes. This response can be measured by assays known to the skilled person, for example ELISPOT (Enzyme-Lynked ImmunoSpot), by intracellular cytokine staining assay, by tetramer assay, by detecting antigen-specific response, by tracking cell multiplication assays, and the presence of markers of cellular activation (CD25, CD69) detected by flow cytometry. The cytotxic ability of CD8⁺ T cells activated with tiT lymphocytes can also be quantified .

The lymphocyte of the present invention can be used in combination with other treatments known to those skilled in the art, for example treatment with antibodies against CTLA-4, PD-1, PDL-1 as well as radiotherapy or chemotherapy.

A fifth aspect of the present invention relates to a kit or device comprising the lymphocyte of the first aspect of the invention or the cell population of the second aspect of the invention.

A sixth aspect of the invention relates to the use of the kit or device of the fifth aspect of the invention for the prevention or treatment of tumor and/or stimulating the immune response against a tumor antigen of any tumor, for example, but not limited to this list: melanoma, lymphoma, chronic lymphocytic leukemia, myeloma, breast, ovary, uterus, cervix, testis, prostate, colon, colorectal, pancreatic , stomach and gastrointestinal tumors, gastric cancer, liver tumor, kidney (comprised clear-cell renal-cell carcinoma), bladder, mouth cancer, pharynx, larynx, esophagus, lung (small and non-small cell), thyroid, glioblastoma, glioma, sarcoma, encephalon, brain, neuroblastoma and marrow, head and neck blastoma, bone and connective tissue. Preferably is melanoma.

The kit of the invention comprises the elements necessary to carry out the methods described herein. The kit may also contain the elements necessary to assess the immune response generated by the lymphocyte of the invention. This kit may include, without any limitation, buffers, enzymes, agents to prevent contamination, etc. It can also include supports and containers required for commissioning and optimization. Also it may further contain positive or negative controls. It may also include instructions to carry out their use.

A seventh aspect of the invention relates to an *in vitro* method to transinfect a lymphocyte comprising the following steps:
a. Isolating antigen-presenting cells, preferably dendritic cells, preferably from peripheral blood, from the patient (for example by adding GM-CSF and IL-4) and isolate lymphocytes, preferably B lymphocytes and/ or CD4⁺ T cells from a biological sample;
b. Differentiating the antigen presenting cell (APC);
c. Infecting these antigen presenting cells from step (b) with a bacterium, wherein the bacterium comprises a tumor peptide;
d. Put into contact the antigen presenting cells from step (c) with the lymphocytes from step (a) at 35-38°C, preferably at 37°C for several hours, preferably 24-72 hours, more preferably 48h, to facilitate the transinfection of lymphocytes.

In another particular embodiment further comprises a step (e) isolation of transinfected lymphocytes.

In a preferred embodiment of the seventh aspect of the invention the bacteria can be both Gram + and Gram -, and also the bacteria can be pathogenic and nonpathogenic. In a more preferred embodiment the bacterium is selected from the list consisting of: *L. monocytogenes, Salmonella enterica,* bacteria from the *Mycobacterium tuberculosis* complex, *Staphylococcus aureus and Escherichia coli*; preferably *L. monocytogenes.* Among the bacteria from the *Mycobacterium tuberculosis* complex can be *M*. *tuberculosis, M. bovis (BCG), M. africanum, M. caprae, M. pinnipedii, M. canetti* y *M. microti.* Preferably, *L. monocytogenes.*

In another preferred embodiment of the seventh aspect of the invention the bacterium comprises a tumor antigen. The tumor antigen may be a tumor which is selected from the list consisting of: melanoma, lymphoma, chronic lymphocytic leukemia, myeloma, breast, ovary, uterus, cervix, testis, prostate, colon, colorectal, pancreatic , stomach and gastrointestinal tumors, gastric cancer, liver tumor, kidney (comprised clear-cell renal-cell carcinoma), bladder, mouth cancer, pharynx, larynx, esophagus, lung (small and non-small cell), thyroid, glioblastoma, glioma, sarcoma, encephalon, brain, neuroblastoma and marrow, head and neck blastoma, bone and connective tissue

In another preferred embodiment of the seventh aspect of the invention the lymphocyte is a heterologous or autologous cell.

The term "in vitro" refers to the method of the invention that is carried out outside of the subject's body. That is, it is done in a biological sample from a subject.

The term "biological sample" herein refers to any sample comprising lymphocytes (CD4⁺T lymphocytes and/or B lymphocytes) and includes, without being limited to, biological fluids or tissues from an individual, obtained by any method known by one skilled in the art that serves for this purpose. The biological sample may be, for example, but not limited, a fluid sample such as blood or serum. The biological sample herein may be fresh or frozen.

The term "tumor antigen" and "tumor peptide" is used interchangeably.

Another embodiment of the present invention relates to a method for the prevention and/or treatment of a tumor and/or stimulating the immune response against a tumor antigen characterized by comprising the administration of a therapeutically effective amount of the pharmaceutical composition of the invention. Preferably the tumor is selected from the list consisting of: melanoma, lymphoma, chronic lymphocytic leukemia, myeloma, breast, ovary, uterus, cervix, testis, prostate, colon, colorectal, pancreatic , stomach and gastrointestinal tumors, gastric cancer, liver tumor, kidney (comprised clear-cell renal-cell carcinoma), bladder, mouth cancer, pharynx, larynx, esophagus, lung (small and non-small cell), thyroid, glioblastoma, glioma, sarcoma, encephalon, brain, neuroblastoma and marrow, head and neck blastoma, bone and connective tissue. Preferably the tumor has neo-antigens. In particular in melanoma several antigens that stimulate the immune system for example are known, but not exclusively to tyrosinase, gp75, gp100, Melan A/MART-1, TRP-2, MAGE family proteins *(melanoma-associated antigen):* GAGE and BAGE, NY-ESO-1, mutations in CDk4 and, β-catenin, MUM-1, p15.

In the present invention the terms "subject", "individual" and "patient" are used interchangeably.

Herein, including the attached claims, the singular forms of words such as "a", "an" and "the" include their corresponding plural referents unless the context clearly indicates otherwise.

Throughout the description and claims the word "comprises" and its variants are not intended to exclude other technical features, additives, components or steps. For those skilled in the art, other objects, advantages and features of the invention will become apparent in part from the description and partly from the practice of the invention. The following examples and figures are provided by way of illustration, and are not intended to be limiting of the present invention.

### BRIEF DESCRIPTION OF THE FIGURES

**FIG. 1****. Transinfected CD4⁺ T cells (tiCD4⁺ T) present bacterial antigens via MHC-I to CD8⁺T cells *"naïve" in vitro.* a,** CD4⁺ tiT transinfected with *Listeria-WT* (wild type strain; top panels) or *Listeria-OVA* (ovalbumin expressing strain; lower panels) were incubated with CD8⁺ *naive* T cells OT-I mice (which recognize a peptide of ovalbumin; OVAp-I 257-264; SIINFEKL (SEQ ID NO: 1) in the context of H-2K^{b}). The histograms represent fluorescence of CD8⁺ T cells labeled with "CellTrace™ Violet". Cell division, a feature of activated cells, is observed as a leftward population (in each cell division dye is diluted). Gray filled histograms show fluorescence of nonactivated *naive* CD8⁺ T cells. Proliferation of CD8⁺ T cells was analyzed at 24h, 48 and 72 after the contacts between CD4⁺ tiT / CD8⁺ cells. **b,** Histograms represent the expression of CD25 or CD69 of CD8⁺T cells incubated with *Listeria-WT* (fine line) or *Listeria-OVA* (thick black line). Gray filled histograms show fluorescence of nonactivated *naive* CD8⁺ T cells. **c,** Histograms show the proliferation of CD8⁺ T cells activated with: anti-CD3 and CD28 antibodies, DCs loaded with soluble pOVA, DCs infected *Listeria-OVA* or tiCD4⁺ T cells transinfected with *Listeria* -OVA to day 2 or day 3. Note that the activation of CD8+ T cells is much greater when activated with tiCD4⁺ T transinfected with *Listeria* -OVA that when activated with DCs infected with *Listeria-OVA.* Histograms filled with gray show fluorescence of nonactivated CD8⁺ T cells. **d,** Histograms represent fluorescence (CellTrace™ Violet) of CD8⁺ T cells from C57BL/6 WT mice or OT-I mice after conjugation with tiCD4⁺ transinfected with *Listeria-OVA.*

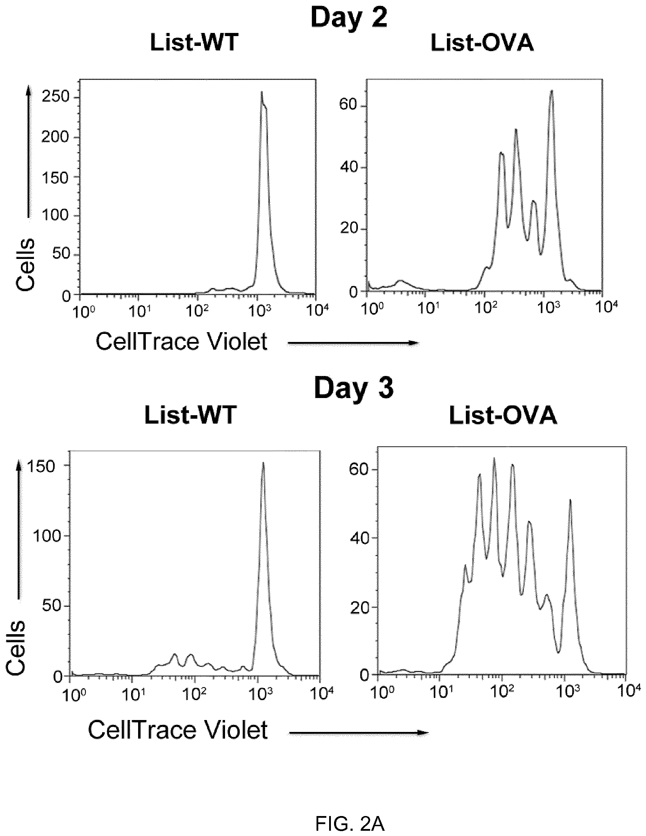

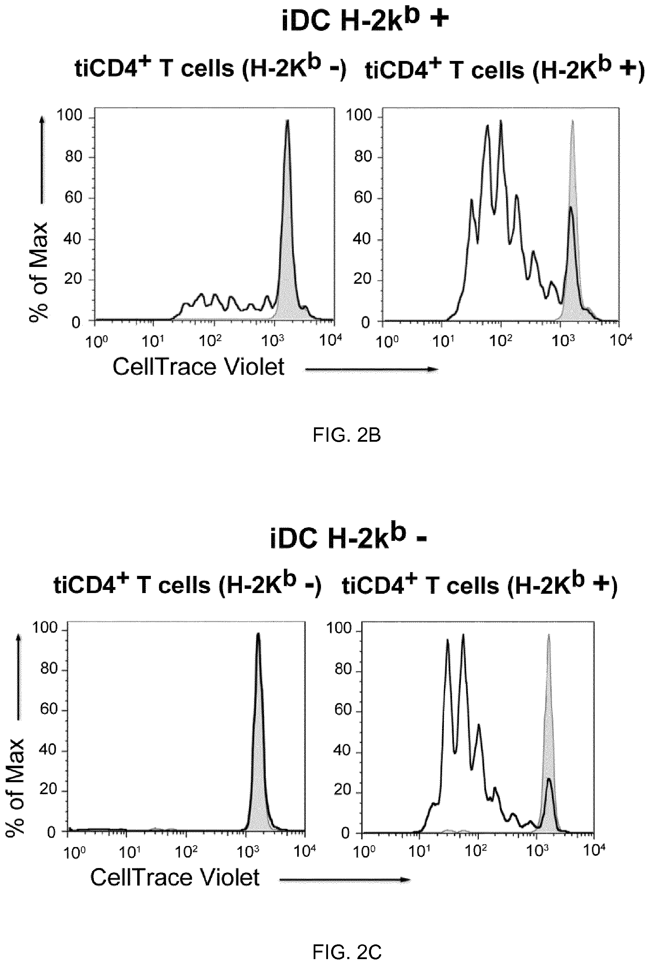

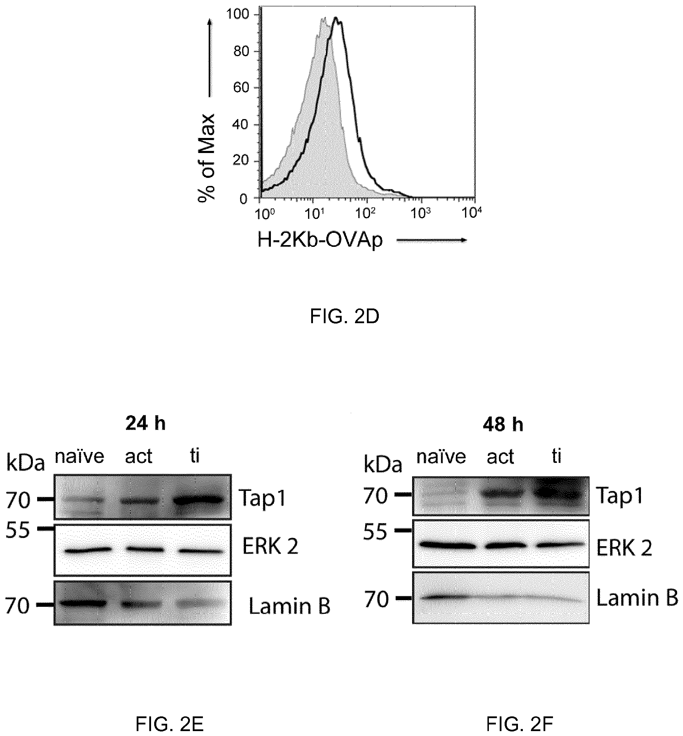

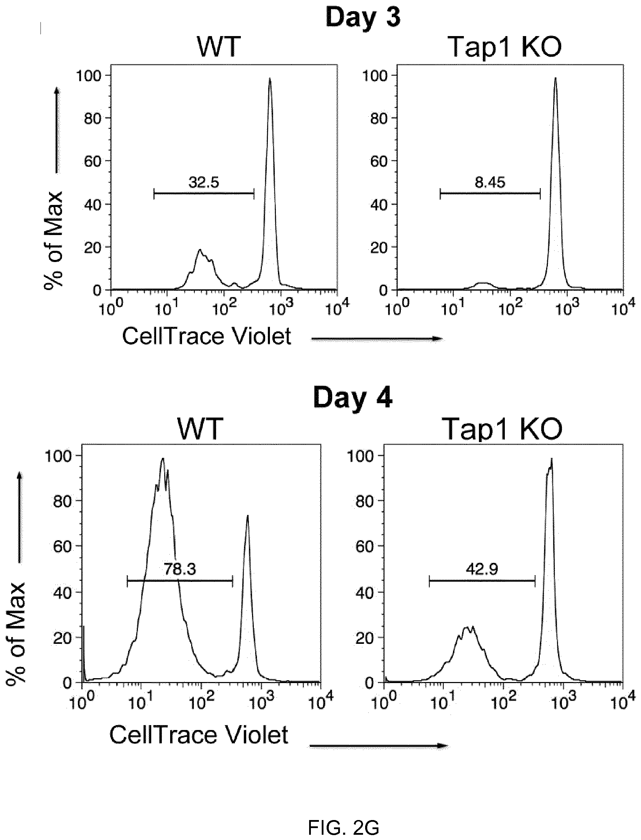

**Fig 2****. tiCD4⁺ T cells process bacterial antigens. a,** Proliferation of CD8⁺ T cells from mice OT-I, 2 or 3 days after incubation with CD4⁺ tiT transinfected with *Listeria* -WT or *Listeria-OVA* cells, which were captured from DCs loaded with the OVA peptide (OVAp-I). **b,** Proliferation of CD8⁺ T cells from mice OT-I incubated with CD4 tiT⁺ cells (H-2K^{b-} or H-2K^{b+}) transinfected with *Listeria-OVA* captured from H-2K^{b+} DCs, or incubated with tiCD4⁺ T cells transinfected with *Listeria*-WT (gray). Note that these CD8⁺ T cells can only be activated when antigens are presented in H2K^{b} (b+) haplotype of MHC-I. **c,** as in **b,** except transinfection, which is made from DCs that were H-2K^{b-} (H-2K^{k/k}), and the conjugates were allowed to form during 48h.**d**, Expression of H-2K^{b}/OVA (detected using a specific antibody) by tiCD4⁺ T cells (H-2K^{b+},black line, H-2K^{b-}, gray) transinfected with *Listeria-OVA* that were captured by DCs from H-2K^{b-}. **e, f,** Western blots showing the expression of Tap1 in *naive* CD4⁺ T cells, activated with DC loaded with soluble OVAp, or with tiCD4⁺ T cells transinfected with *Listeria-OVA* for 24 (E) or 48 h (F) after activation. ERK2 and laminB were used as controls. **g,** CD8⁺ T cell proliferation from mice OT-I, 3 or 4 days after conjugation with tiCD4⁺ T cells (from Tap1 KO mice, or its isogenic WT) transinfected with *Listeria-*OVA.

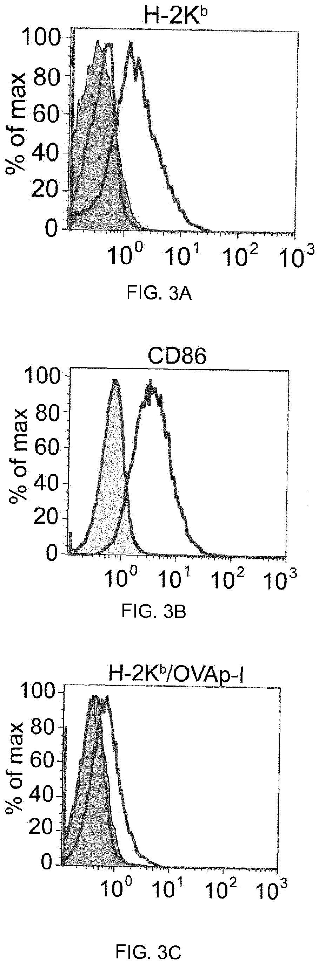

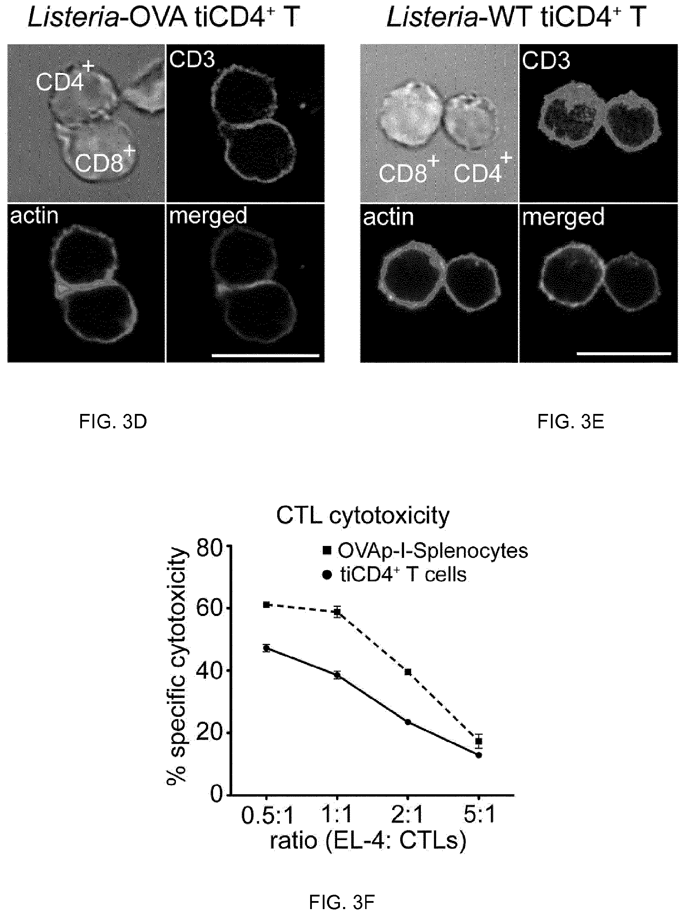

**FIG. 3****. tiCD4⁺ cells form immune synapses with *naïve* CD8⁺ T cells. a**, Histogram shows H-2K^{b} expression in CD4⁺ T cells before (black line) and after transinfection (gray line). Filled gray histogram shows the fluorescence of the negative control. **b,** Expression of CD86 on CD4⁺ T cells before (black line) and after transinfection (gray line). Filled gray histogram shows the fluorescence of the negative control. **c,** Expression of H-2K^{b}/OVAp-1 in tiCD4⁺ T cells transinfected with *Listeria-OVA,* 24 (line does not move) or 48 h (line moving right) post-transinfection and non-transinfected T cells (filled gray histogram). **d** and **e**, confocal images of tiCD4⁺ T cells transinfected with *Listeria*-WT **(d)** and *Listeria-OVA* **(e)** incubated with CD8⁺ *naive* T cells from OT-I mice for 1 h. It is shown CD3 fluorescence and actin. Bars represent 10 µm. **f**, Relative specific cytotoxicity of CD8⁺ T effector cells (CTLs) activated by tiCD4⁺ T transinfected with *Listeria-OVA* (solid line) or splenocytes loaded with pOVA (dotted line). The specific cytotoxicity was measured using different ratios of EL-4 (target cells): CTLs (0.5: 1, 1: 1, 2: 1, 5: 1).

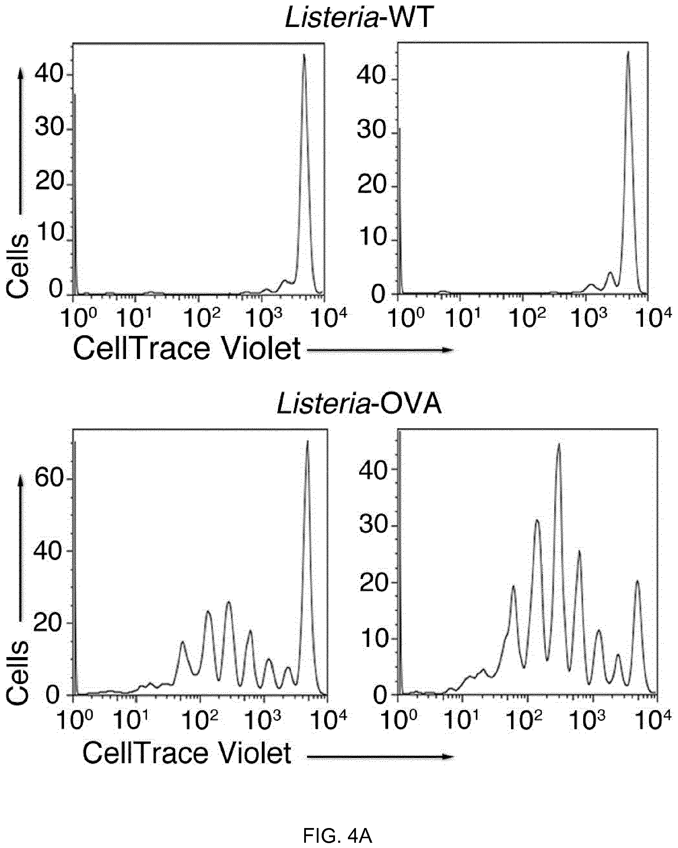

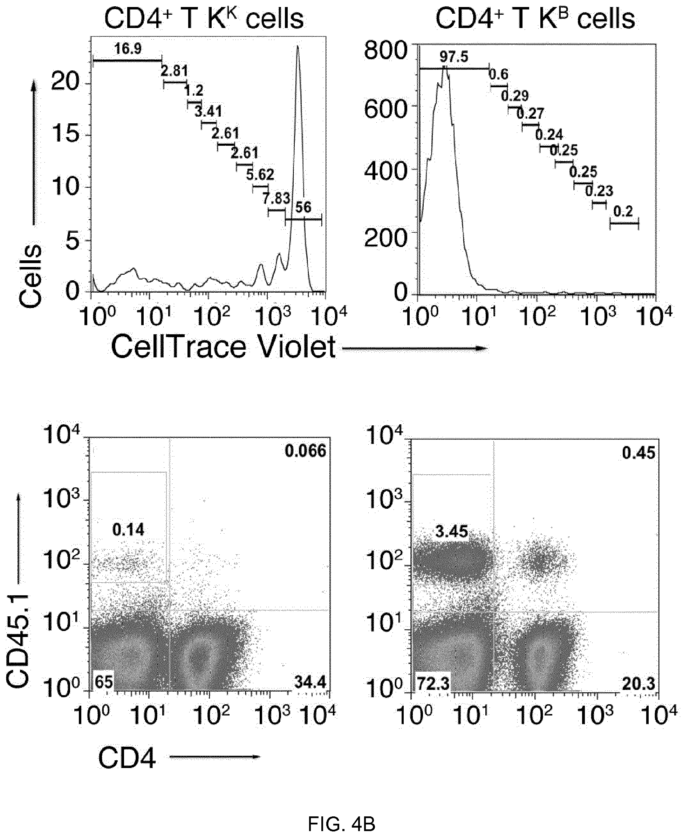

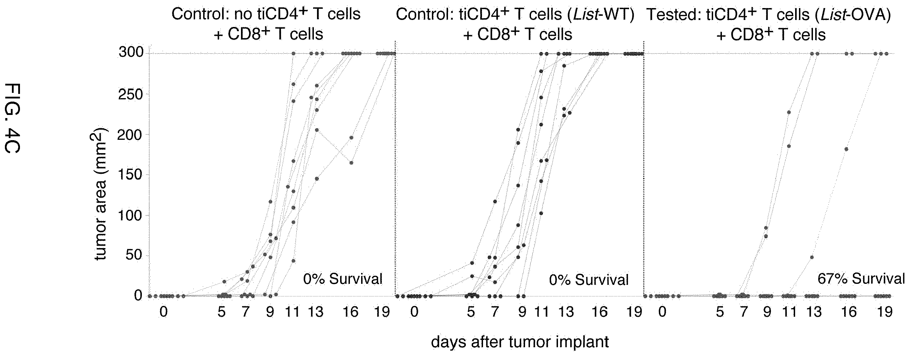

**FIG. 4****. tiCD4⁺ cells activate cytotoxic effector T cells *in vivo. a*,** Histograms show fluorescence *(CellTrace* ™ *Violet)* of CD8⁺ CD45.1⁺ T cells injected in receptor mice (CD45.2), which were inoculated with tiT CD4⁺ cells transinfected with *Listeria*-WT or *Listeria-OVA.* 2 x 10⁶ tiCD4⁺ cells (left panels) or 5 x 10⁶ tiCD4⁺ cells (right panels). **b,** *In vivo* proliferation of CD8⁺CD45.1⁺ T Cells, from OT-I mice, stained with CellTrace Violet injected in wild type C57BL/6 recipients (whose bone marrows have been reconstituted after irradiation with cells from H-2K^{k} mice). CD8⁺ CD45.1⁺ T cells from OT-I mice (4 x 10⁶ cells/mouse) were transferred along with CD4⁺ T cells expressing H-2K^{b} (right panels) or H-2K^{k} (panels left) from AND mice (4 x 10⁶ cells/mouse) and MCC peptide (15 µg/mouse). Mice also were infected with *Listeria-OVA* (10³ bacteria/mouse). 5 days after infection, spleens were isolated and proliferation of transferred CD8⁺ T cells was detected by flow cytometry (panels above). Panels below show CD8⁺ T cells transferred from OT-I mice (CD45.1⁺ CD4⁻). **c,** (5 x 10⁵) B16 OVA melanoma cells (ovalbumin expressing) were injected subcutaneously into the right flank of recipient mice. All groups (9 mice/group) were inoculated with CD8⁺ *naive* T cells from OT-I mice (10³/mouse) together with phosphate buffered saline (PBS) in Group 1; 5x10⁵ tiCD4⁺ cells transinfected with *Listeria* WT, in Group 2; 5x10⁵ tiCD4⁺ transinfected with *Listeria-OVA* in group 3. The size of the tumors was monitored every two days (from day 5 to 13) and every 3 days thereafter.

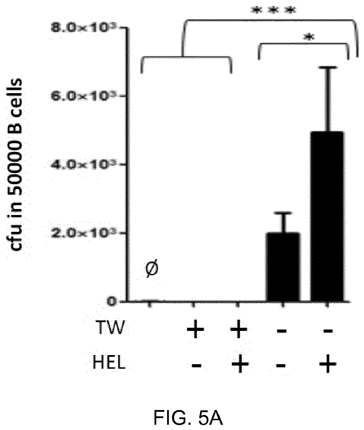

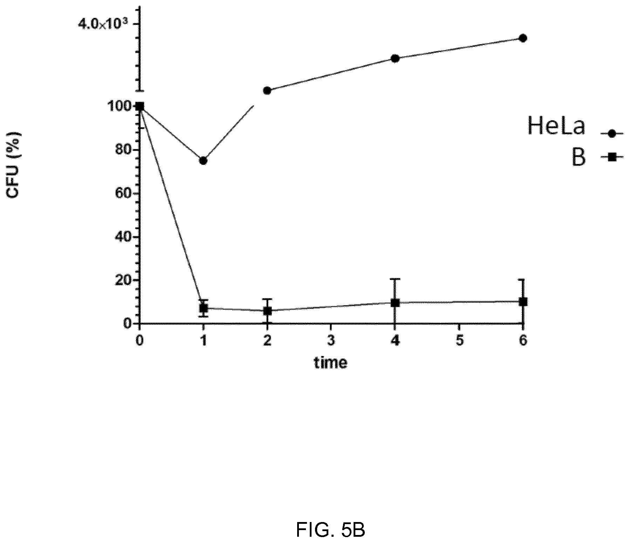

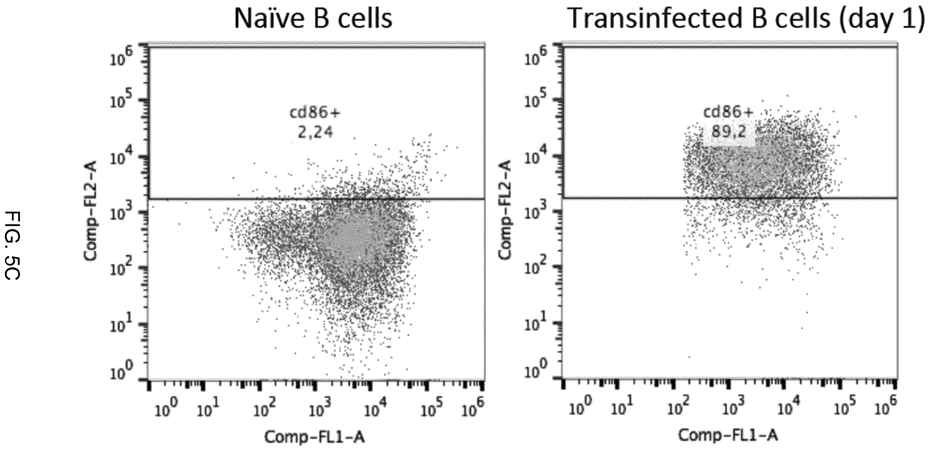

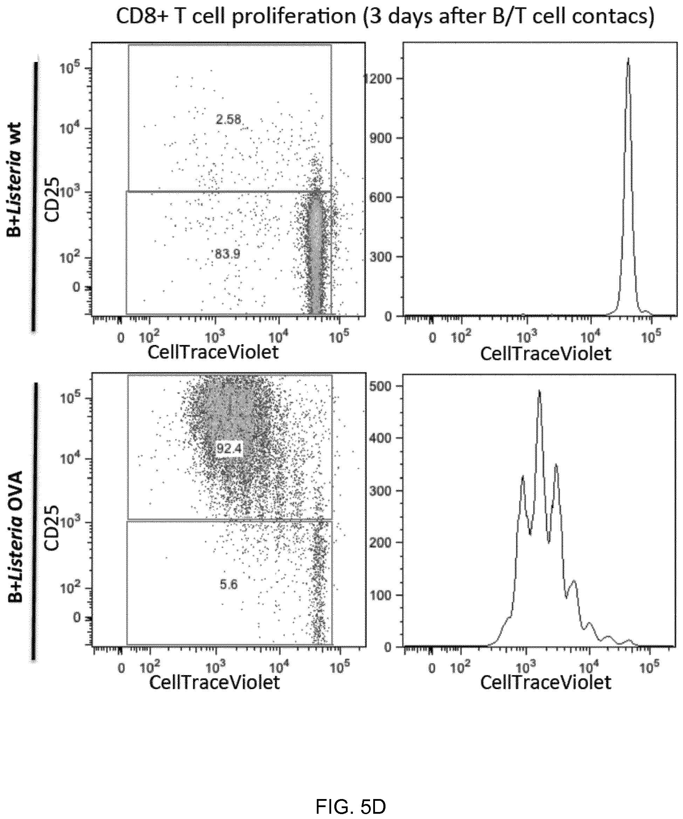

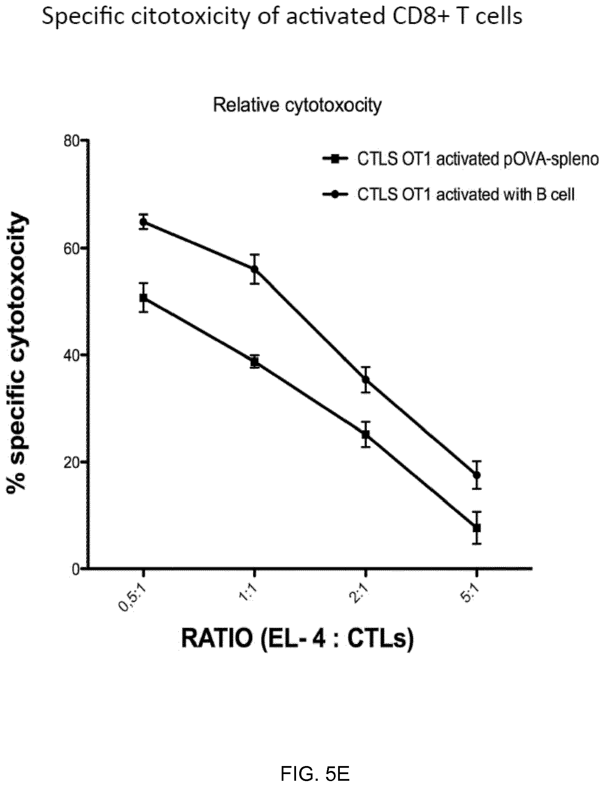

**FIG. 5****. B cells capture bacteria by transinfection and cross-present bacterial antigens. a,** Murine DC decorated + (or not -) with Hen Egg Lysozyme (HEL) were infected with *L. monocytogenes,* and incubated with B cells from MD4 transgenic mice, whose B cells have B cell receptors (BCR) recognizing HEL, under conditions that allow the DC-B cell interactions (for 30 min), or in the presence of a polycarbonate barrier (transwell) which prevents such interactions. After formation of the conjugates, CD4⁺ T cells were re-isolated and conducted a classic gentamicin survival assay. CFU (colony forming unit; intracellular bacteria) are shown for each 50,000 CD4⁺ T cells. Ø is direct infection of bacteria on B cells. **b,** DC infected with *L. monocytogenes* and decorated with HEL were incubated with B cells from MD4 mice. After formation of conjugates, B cells were re-isolated and it was quantified the survival (at different times) of intracellular bacteria by gentamicin resistance assays. Data were normalized to time 0. As control of bacterial fitness HeLa cells were infected in parallel. **c**, Expression of CD86 on B cells before (left panel; *naive* B cells) and one day after transinfection with *Listeria-OVA* (right panel). In both panels is shown IgM expression (FL-1 channel) vs expression of CD86 (channel FL-2). **d**, Proliferation of *naive* CD8⁺ T cells from OT-I mice labeled with CellTrace Violet, after 3 days of contact with tiB cells that have captured by transinfection *Listeria*-WT (top panels) or *Listeria-OVA* (below). Left panels show CellTrace Violet fluorescence vs CD25 expression (a marker of T cell activation). **e,** Specific relative cytotoxicity of CD8⁺ T cells (CTL) activated by tiB cells transinfected with *Listeria-OVA* (top line) or splenocytes decorated with OVAp-I peptide (bottom line). Different ratios of EL-4(target cells):CTL were measured.

### EXAMPLES

Below, the invention will be illustrated by assays performed by the inventors, which demonstrate the effectiveness of the product of the invention.

The fascinating discovery that T cells can capture bacteria from DCs infected through a process called transfection (Cruz-Adalia *et al.,* 2014), prompted us to ask whether it was possible that transinfected T cells (ti) could act as true APCs. This was a risky question because, if true, it will break a dogma of immunology, the strict separation of roles between innate and adaptive immunity, and would be a huge advance in the basic understanding of how the immune system works.

To answer this question, we generated pure populations (by cell sorter) of CD4⁺ T cells transinfected (hereinafter called cells "tiCD4⁺ T") with *Listeria-OVA* (*L. monocytogenes* expressing ovalbumin) or *Listeria-* WT (wild isogenic strain not expressing OVA) by using dendritic cells from bone marrow (BM-DCs) previously infected with the bacteria. The ability to present antigens of tiCD4⁺ T cells was tested by incubating them with naive CD8⁺ T cells isolated from transgenic OT-I mice. These CD8⁺ T cells recognize a peptide of ovalbumin (OVAp-I 257-264; SIINFEKL, SEQ ID NO: 1) in the H-2K^{b} context. The flow cytometric analysis of CD8⁺ T cells stained with "CellTrace ™ Violet" shows a potent proliferation of CD8⁺ T cells that begins two days after contact with the tiCD4⁺ T cells (a movement to the left observed of the "CellTrace ™ Violet" fluorescence), but only in those CD8⁺ T cells that were in contact with CD4⁺ T cells transinfected with *Listeria-OVA* (Fig 1a). This highly proliferative population expresses high levels of CD8 (CD8^{+ high}) (Fig. 1a). However, when CD8⁺ T cells were incubated with tiCD4⁺ T cells transfected *Listeria-WT* they do not proliferate (Fig. 1a). According to these data, the expression of the activation markers CD69 and CD25 was detected only in CD8⁺ T cells incubated with tiCD4⁺ T cells transinfected with *Listeria-OVA* but not when incubated with tiCD4⁺ T cells transinfected with *Listeria-WT* (Fig. 1 B). tiCD4⁺ T cells transinfected with *Listeria-OVA* induced the proliferation of CD8⁺ T cells in a more potent way than the induced by BM-DCs loaded with soluble OVA peptide, or even the proliferation induced by polyclonal activation with CD3/CD28 antibodies, and it was far more stronger than the produced by BM-DCs infected with *Listeria-OVA* (Fig. 1c). tiCD4⁺ T cells transinfected with *Listeria-OVA* on other hand are not able to induce proliferation of CD8⁺ T cells from wild mice, which do not recognize any OVA antigen (Fig. 1d), whereas they induce very potent proliferation of CD8⁺ T cells from OT-I mice (Fig. 1a, c, d), indicating that the observed effects are antigen-specific.

Taken together, these data suggest that tiCD4⁺ T cells are able to cross-present bacterial antigens to *naive* CD8⁺ *T cells.* At this point, we wondered to what extent tiCD4⁺ T cells could recapitulate different stages of the cross-presentation given in professional APC: endogenous antigen processing, antigen presentation via MHC-I, expression of co-stimulatory molecules, and interaction with target T cells through the formation of canonical immune synapses.

Antigen presentation of tiCD4⁺ T cells could be due to the capture of the MHC/antigen complexes from the surface of the DCs or by endogenous processing of the tiCD4⁺ cells themselves. To distinguish between these two options, CD4+ T cells were transinfected with *Listeria-WT* or *Listeria-OVA* from BM-DCs, previously loaded with the OVA peptide (OVAp/OT-I; cells that recognize CD8⁺ T cells from OT-I mice); later this tiCD4⁺ T cells (re-purified) were used to stimulate *naive* CD8⁺ T cells. In this configuration, antigen capturing from DCs cells would lead to a comparable activation of CD8⁺ T cells in both experimental conditions. However, it is observed that tiCD4⁺ cells transinfected with *Listeria-OVA,* but not with *Listeria*-WT induce a very strong activation of CD8⁺ T cells (Fig. 2a). Additional experiments using tiCD4⁺ T cells from transgenic mice expressing only the haplotype H-2K^{k} (MHC-I), and therefore not able to stimulate CD8⁺ T cells from mice OT-I or their isogenic tiCD4⁺ T cells that express H-2K^{b}, capturing both cell types bacteria from DCs expressing H-2K^{b}, confirm that activation of CD8⁺ T cells is due in its vast majority by antigen processing inside tiCD4⁺ T cells (Fig. 2b).

A possible contribution to antigen presentation by the BM-DCs (eg contamination) was totally excluded by incubating CD8⁺ T cells from OT-I mice with CD4⁺ T cells (H-2Kb or H-2Kk) transinfected with *Listeria-OVA* from DCs expressing H-2K^{k} only. Exclusively tiCD4⁺ T cells expressing H-2K^{b} induced proliferation in CD8⁺ T cells (Fig. 2c) presenting OVA antigens in their MCH-I H-2K^{b} molecules (Figure 2d). Western blot analysis revealed in turn that CD4⁺ T cells activated and transinfected increase the expression of Tap1 (Fig. 2e, f), a protein involved in antigen presentation via MHC-I. Moreover, tiCD4⁺ T cells isolated from Tap1 KO mice showed very reduced capacity as cross-presenting cells after bacterial capture (Fig. 2g), ie, the proliferation of CD8⁺ T cells was greatly reduced when antigen presenting cells were tiCD4⁺ from mice Tap1 KO compared to that observed when presenting cells were tiCD4⁺ from WT mice.

It was also found that after transinfection, tiCD4⁺ T cells increased the expression of MHC-I (H-2K^{b}), H-2K^{b} coupled to OVA antigen (of bacterial origin) and ligands of co-stimulatory molecules, such as CD86 (Fig. 3a-c), which supports a canonical antigen presentation on MHC-I. Activation of CD8⁺T cells involved the formation of CD4⁺/CD8⁺ cell conjugates Fig. 1c), indicative of the formation of the immune synapse (IS), mark of T cell activation by APCs. The structure of a mature IS is composed of multimolecular concentric rings called supramolecular activation complexes (SMACs). Central SMACs (cSMAC) contain the MHC-TCR complex, and the peripheral zone (pSMAC) forms a ring structure containing adhesion molecules and F-actin (Saito and Batista, 2010). Immunofluorescence analysis confirms that *naive* CD8⁺ T cells form mature IS with tiCD4⁺ T cells transinfected with *Listeria-OVA* (Fig. 3e). CD4⁺/CD8⁺ cell conjugates contain CD3 molecules, part of the TCR complex, recruited in cSMAC, and show a massive accumulation of actin in the pSMAC; we found no evidence of the formation of these structures when using tiCD4⁺ T cells transinfected with *Listeria-WT* (in the few cell contacts that are detected) (Fig. 3d). Together, these data demonstrate that tiCD4⁺ T cells are professional APCs and activate CD8⁺ T cells by canonical cross-presentation.

Activation of CD8⁺T cells by tiCD4+ T cells transinfected with *Listeria-OVA* gives them cytotoxic capacity because they can eliminate EL-4 target cells expressing OVAp/OT-I (Fig. 3f). Furthermore we investigated whether tiCD4⁺ T cells are able to activate *naive* CD8⁺ T cells *in vivo.* For this purpose *naive* CD8⁺ T cells were isolated from OT-I/CD45.1 mice, stained *ex vivo* with "CellTrace ™ Violet", transferred to a BL6 / CD45.2 mouse, and tiCD4⁺ T cells were injected to elicit a response. We found that CD45.1 CD8⁺ T cells isolated from the spleen proliferate in response to tiCD4⁺ T cells transinfected with *Listeria-OVA* but not if they were transinfected with *Listeria-WT* (Fig. 4a). Moreover, we analyzed whether CD4⁺ T cells cross-present antigens *in vivo* during physiological activation of the immune system. To do so, the bone marrows of receptors C57BL/6 mice were transplanted with stem cell progenitors with haplotype H-2K^{k} (APCs from these mice will not be able to cross-present antigens to CD8⁺ T cells from OT-I mice). One month later, CD4⁺ T cells from AND mice (H-2K^{b} or H-2K^{k}) were transferred in recipient mice together with CD45.1⁺CD8⁺ T cells from OT-I mice, and MCC peptide (to favor transinfection (Cruz-Adalia et al., 2014)). The recipient mice were also intravenously infected with *Listeria-OVA.* Only mice that received CD4⁺ T cells H-2K^{b} were able to activate OT-I CD8⁺ CD45.1⁺ T cells (Fig. 4b), confirming the capacity for presenting antigens of CD4⁺ T cells during the course of a bacterial infection.

To further explore the therapeutic potential of CD8⁺ T cell activation by tiCD4⁺ T cells, we tested whether tiCD4⁺ T cells could protect against the formation of tumors using a mice model of aggressive melanoma. B16-OVA melanoma cell line (Borroto *et al.,* 2014) was injected subcutaneously in C57BL/6mice. One day later, *naive* OT-I CD8⁺ T cells were transferred in a single injection together with: Group 1: PBS; Group 2: tiCD4⁺ cells transinfected with *Listeria*-WT; and Group 3: tiCD4⁺ cells transinfected with *Listeria-OVA.* All mice in the control groups, vehicle treated cells or tiCD4⁺ T cells transinfected with *Listeria*-WT, developed tumors during the first 11 days after injection of B16-OVA cells (Fig. 4c). Treatment with tiCD4⁺ T cells transinfected with *Listeria-*OVA prevented tumor formation in 6 out of 9 mice and delayed tumor appearance in another mice, ie vaccination (single inoculation) with tiCD4⁺ cells working as antigen presenting cells conferred protection against melanoma in 7 of 9 cases (Fig. 4c). These results show that the cross-presentation by tiCD4⁺ T cells can be used *in vivo* to activate effector CD8⁺ T cells against tumors.

In conclusion, we show for the first time that cross-presentation of bacterial antigens, which was thought to be the exclusive task of APCs of the innate immune system, can be carried out effectively by CD4⁺ T cells, considered as the paradigm of the adaptive immune system. We also show evidence that this new way of antigen presentation can be exploited as a tool for cancer immunotherapy. The antitumor activity of tiCD4⁺ T cells is remarkable; note that a single injection provides protection against B-16, an extremely aggressive melanoma, whereas vaccines based on DCs require multiple inoculations, and even so they do not achieve such positive effects as model tiCD4⁺ (Mayordomo et al., 1995).

Moreover, we have also shown that B cells are also capable of capturing bacteria by transinfection. The process of bacterial capture by B cells from infected DC was quantitated on reisolating B cells after conjugate formation with infected DC, followed by classic gentamicin survival assays. This method allows us to analyze a large number of conjugates. The requirement of cell-cell contact was tested using a physical barrier (polycarbonate filters) that prevented DC-B cell contacts , and the role of antigen recognition by using DC decorated with hen egg lysozyme (HEL) (note that we use B cells isolated from MD4 transgenic mice, whose B cells have B cell receptors (BCR) t recognizing HEL). The largest uptake of bacteria by B cells was observed when physical contact between DC (infected) and B cells was allowed, and the DC were decorated with HEL (Fig. 5a), which is consistent with a transinfection via immunological synapse and is also similar to what observed in CD4⁺ T cells (Cruz-Adalia et al., 2014). When DC-B cell contact is prevented, bacterial capture by B cells is very low and similar to what has been observed in direct bacterial infections on B cells (negative control). We only took into account the experiments where B cell purity was greater than 97%, and colony forming units (CFUs) from contaminant cells were excluded from the results. To determine the fate of bacteria captured by B cells, HEL-decorated DC were infected with *L. monocytogenes* and conjugated with B cells from MD4 mice. After the formation of conjugates, B cells were reisolated and CFU (intracellular bacteria in B cells) were analyzed by gentamicin survival assays, where B cells were collected at different times. Unexpectedly, B cells killed very quickly (and efficiently) the bacteria captured (Fig. 5b). In the first hours after the formation of conjugates, over 90% of the internalized bacteria were destroyed. As control of bacterial fitness we measured the CFU after infecting HeLa cells (Fig. 5b).

Transinfection of *Listeria-OVA* induces an increased expression of MHC-I (H-2K^{b}), accumulation of H-2K^{b} coupled to OVAp-I antigen (not shown), and the expression of the CD86 co-stimulatory receptor in B cells (Fig 5c.); these results are consistent with a role of antigen cross-presentation by transinfected B cells (tiB).

We generated a tiB cell population from MD4 mice, transinfected with *Listeria* -OVA or *Listeria*-WT. We analyzed the ability to present antigens of tiB cells, incubating them with *naive* CD8⁺T cells isolated from OT-I transgenic mice. Flow cytometric analysis showed a strong proliferation of CD8⁺ T cells 3 days after exposure to the tiB cells (Figure 5d), but only when the tiB had captured *Listeria-OVA.* CD8⁺ T cells incubated with tiB that had captured *Listeria*-WT did not proliferate (Fig. 5d). Expression of activation marker CD25 was detected in CD8⁺ T cells incubated with tiB transinfected with *Listeria-OVA,* but not when used tiB transinfected with *Listeria-WT* (Fig. 5d). The tiB cells transinfected with *Listeria* -OVA induce proliferation of CD8⁺ T cells more potently than even the bone-marrow derived DCs (BM-DC) loaded with soluble peptide OVAp-I, infected with *Listeria-OVA,* or the polyclonal activation of CD8⁺ T cells themselves with anti CD3/CD28 antibodies. We also analyzed the ability of cytotoxic CD8⁺ T cells, and found that this cytotoxic activity was greater when stimulated with tiB cells transinfected with *Listeria-OVA* that when stimulated with splenocytes decorated with OVAp-I peptide (Fig. 5e). Cytotoxic activity was analyzed as the ability to remove EL-4 lymphoma cells decorated with OVAp-I. These data support the hypothesis that B cells behave similarly to the CD4⁺ T cells, capturing bacteria by transinfection and cross-presenting such antigens, inducing a potent activation of CD8⁺ T cells that are responsible for eliminating tumors expressing the recognized antigens .

### Materials and Methods.

Bacteria: *Listeria monocytogenes-OVA* (pPL2-LLO-OVA), a *Listeria* strain that expresses OVA protein, and its WT isogenic strain *L. monocytogenes* 10403S.

Mice: (1) Wild-type C57BL/6 mice, (2) C57BL/6-Tg (TcraTcrb)425Cbn/J OT-II mice expressing a T cell receptor (TCR) specific for OVA peptide 323-339 in the context of MHC class II (I-Ab), (3) C57BL/6-Tg(TcraTcrb)1100Mjb/J OT-I mice expressing TCR specific for OVA peptide 257-264 in the context of H-2K^{b} (4) AND-TCR transgenic mice recognizing moth cytochrome c 88-103 (ANERADLIAYLKQATK) (MCCp) on I-E^{k}, expressing H-2K^{b}, H-2K^{k} or both haplotypes. (**5**) C57BL/6-Tg(IgheIMD4)4Ccg/J, (MD4) transgenic mice with more than 99% of their B cells expressing a B cell Receptor (BCR) specific for hen egg lysozyme (HEL). Male or female mice aged 8 to 12 weeks were used for experiments. Mice were kept in the specific-pathogen-free (SPF) unit at the CNB-CSIC animal facility. Experimental procedures were conducted in accordance with Spanish and European guideline, and under the FELASA guidelines.

### Primary cells:

Dendritic cells (DC) were generated as described in Inaba *et al.,* 1992. Briefly, cells from the bone marrow (from the tibias and femurs of C57BL/6 mice 8-20 weeks) were grown in the presence of GM-CSF, at day 10 cells were tested by flow cytometry for CD11c, IA/IE and Gr1 to ensure that they had differentiated properly. They were matured with LPS day before use.

Primary CD4⁺ T from OT-II mice and CD8⁺ T from OT-I mice were obtained from lymph nodes and spleen by negative magnetic selection, as is described in Cruz-Adalia *et al.,* 2014.

### Cell lines

The EL-4 lymphoma line was maintained in RPMI 1640 (Fisher Scientific), supplemented with 10% FCS, 0.1 U/ml penicillin, 0.1 mg/ml streptomycin (Lonza) and 0.05 mM 2-mercaptoethanol.

Melanoma B16 OVA line was maintained in RPMI 1640 with 0.4 mg/ml geneticin. Antibiotics were washed 48h before inoculation.

### Antibodies:

Antibodies used were anti-CD69, -CD25, -CD4, -CD8, -CD11c, -IA/IE, -Gr1 (BD and Immunostep), biotinylated antibodies against CD45.1, CD3, CD4, CD8, CD28, IgM , B220, CD19, MHC class II (I-Ab), CD11b, CD11c DX5, CD25 and CD16/CD32 (BD and Immunostep), and anti-tubulin FITC-conjugated (Santa Cruz). The monoclonal antibody 25-D1.16 specific for SIINFEKL/H-2K^{b} (SEQ ID NO: 1/H-2K^{b}) and labeled with allophycocyanin (APC) was purchased from eBioscience. Anti-TAP-1 (M-18), - ERK-2 and -lamin B were purchased from Santa Cruz. Secondary antibody duck anti-mouse and goat anti-hamster conjugated with AlexaFluor488, 647, or 568 were purchased from Life Technologies; secondary antibodies peroxidase conjugated with goat anti-mouse IgG and goat anti-rabbit IgG were purchased from Thermo Scientific.

### Reagents

The OVAp/OT-II peptide (OVA 323-339; ISQAVHAAHAEINEAGR, SEQ ID NO: 3) and OVAp/OT-I (OVA 257-264, SIINFEKL (SEQ ID NO: 1)) were generated at the Center for Molecular Biology Severo Ochoa (CBMSO-CSIC). Mouse GM-CSF was from Peprotech. The peptide (MCCp) 88-103 (ANERADLIAYLKQATK; SEQ ID NO: 2) was purchased from GenScript. LPS (Sigma-Aldrich), microparticles coupled to streptavidin (MiltenyiBiotec), streptavidin-PerCP (BD) and Alexa Fluor 568-Phalloidin(Life Technologies). Poly-L-Lysine (Sigma-Aldrich), *Cell*Trace ™ *Violet* (Life Technologies) and 7-AAD *Viability staining solution* of eBiosciences.

### Transinfection of CD4⁺ T cells

CD4⁺ T cells from mice OT-II were transinfected with *Listeria-OVA* or *Listeria-WT* as described in Cruz-Adalia *et al.* 2014. Briefly, BM-DC infected and loaded with OVAp (OT-II) (to increase transinfection) formed conjugates with CD4⁺ T from OT-II mice. In some experiments the BM-DC and CD4⁺ T cells were isolated from AND mice expressing MHC-I haplotypes: H-2K^{b/k}, or H-2K^{b} negative, H-2K^{k/k}. In these experiments the BM-DC were loaded with MCCp to enhance transinfection. After 3h of conjugates DC/T it was added gentamicin (100 µg/ml) to the medium to remove extracellular bacteria. 24h later (in some experiments 48h) the tiCD4⁺ cells purified by cell sorting (FACS Synergy; iCyt).

### Proliferation Assays of CD8⁺ T

The purified population of tiCD4⁺ cells was incubated with *naive* CD8⁺ T cells isolated from OT-I mice. These CD8⁺ T cells previously stained with *CellTrace* ™ *Violet* to quantify their proliferation by flow cytometry (FACSAria; BD). In each cell division fluorescence is lost in the proliferating population, and it was observed as a leftward shift in the histogram. Only the living cells, which do not capture the dye 7AAD, were analyzed.

To analyze the proliferation of CD8⁺ T cells *in vivo,* 5x10⁶ *naive* CD8 ⁺ T cells (from mice CD45.1⁺ OT-I), stained with *CellTrace* ™ *Violet,* were injected intravenously into recipient mice (CD45.2⁺ C57BL/6). After 24h, the tiCD4⁺ cells were transferred to mice. Spleens were isolated after three days to analyze the proliferation of CD8⁺ T cells by flow cytometry.

### Fluorescence microscopy:

tiCD4⁺ and *naive* CD8⁺ T cells were allowed to form conjugates for 1 h, then fixed with 4% paraformaldehyde in PBS. CD8+ T cells were previously stained with *CellTrace* ™ *Violet.* Samples were permeabilized with 0.1% Triton ™ X-100 in PBS prior to staining with the indicated antibodies. F-actin was detected using phalloidin coupled to a fluorophore. Samples were visualized by confocal microscopy using a Leica TCS SP5 equipped with 63x-lenses and controlled by Leica LAS AF. Images were analyzed using ImageJ (NIH Bethesda, MD).

### Cytotoxicity assay

CD8⁺ T cells both effector and cytotoxic (CTLs) were prepared from naïve CD8⁺ cells cells (OT-I) activated with tiCD4⁺ transinfected with Listeria-OVA. As positive controls were used CD8⁺ T cells from OT-I activated with splenocytes loaded with OVAp.

EL-4 cells were incubated with 0.5 µM OVAp/OT-I for 1 h (or not, as control). After PBS washes, EL-4 cells loaded with OVAp were stained with CellTrace ™ Violet (5 µM). On the other hand, EL-4 cells without loading OVAp were stained with CellTrace ™ Violet (0.5 µM). After washing with culture medium both populations were mixed and incubated with different ratios of CTLs (5:1, 2:1, 1:1, 0.5:1) (EL4/CTLs). After 4h, samples were analyzed by flow cytometry. To calculate the specific cytotoxicity it was employed the following formula: 1 - (% of EL-4 Cell Violet^{high} / % of EL-4 Cell Violet^{low}) x 100 (Lang et al., 2005). Cytotoxicity is calculated relative subtracting the negative control (EL-4 cells not incubated with CTLs).

### Protection assay against tumors (B16-OVA melanoma)

CD4⁺ T cells transinfected with *Listeria-WT* (as negative control) or *Listeria-OVA* were prepared as described in previous sections. 24h after transinfection, tiTCD4⁺ are re-isolated cell sorting and resuspended in PBS. *naive* CD8⁺ T cells from OT-I were purified by magnetic columns as previously described (Cruz-Adalia *et al.,* 2014) and resuspended in PBS. 5 x 10⁵ B16-OVA cells were injected subcutaneously into the right flank of recipient mice (C57BL/6). After that, mice were divided into three groups and transferred intravenously (iv) in a single injection: Group 1: PBS; Group 2: 5x10⁵ tiCD4⁺ cells transinfected with *Listeria-WT;* and Group 3: tiCD4⁺ cells transinfected with *Listeria-OVA.* All mice in all groups were also injected, simultaneously to tiCD4⁺ or PBS vehicle, with 10³ CD8⁺ T cells from OT-I (also i.v.). Tumor growth was monitored every 2-3 days using a caliper-dial, and the areas were determined by multiplying the length and width. The experimental groups were assigned randomly and the experiment was conducted in a double blind (people who measured the tumors did not know to which group each mouse belonged). Mice were sacrificed when tumors reached 300 mm² according to the criteria endpoint as directed by the European Union and FELASA for experimental animals, and in accordance with the current legislation in Spain.

### BIBLIOGRAPHY:

Borroto, A., Arellano, I., Blanco, R., Fuentes, M., Orfao, A., Dopfer, E.P., Prouza, M., Suchànek, M., Schamel, W.W., and Alarcón, B. (2014). Relevance of Nck-CD3 epsilon interaction for T cell activation in vivo. J. Immunol. 192, 2042-2053.
Chen, D.S., and Mellman, I. (2013). Oncology Meets Immunology: The Cancer-Immunity Cycle. Immunity 39, 1-10.
Clarke, S.R., Barnden, M., Kurts, C., Carbone, F.R., Miller, J.F., and Heath, W.R. (2000). Characterization of the ovalbumin-specific TCR transgenic line OT-I: MHC elements for positive and negative selection. Immunol. Cell Biol. 78, 110-117.
Cruz-Adalia, A., Ramirez-Santiago, G., Calabia-Linares, C., Torres-Torresano, M., Feo, L., Galán-Díez, M., Fernandez-Ruiz, E., Pereiro, E., Guttmann, P., Chiappi, M., et al. (2014). T Cells Kill Bacteria Captured by Transinfection from Dendritic Cells and Confer Protection in Mice. Cell Host Microbe 15, 611-622.
Inaba, K., Inaba, M., Romani, N., Aya, H., Deguchi, M., Ikehara, S., Muramatsu, S., and Steinman, R.M. (1992). Generation of large numbers of dendritic cells from mouse bone marrow cultures supplemented with granulocyte/macrophage colony-stimulating factor. J. Exp. Med. 176, 1693-1702.
Joffre, O.P., Segura, E., Savina, A. and Amigorena, S. (2012). Cross-presentation by dendritic cells. Nat. Rev. Immunol. 12, 557-569.
Kalos, M., and June, C.H. (2013). Adoptive T Cell Transfer for Cancer Immunotherapy in the Era of Synthetic Biology. Immunity 39, 49-60.
Lang, D., Lu, M.M., Huang, L., Engleka, K.A., Zhang, M., Chu, E.Y., Lipner, S., Skoultchi, A., Millar, S.E., and Epstein, J.A. (2005). Pax3 functions at a nodal point in melanocyte stem cell differentiation. Nature 433, 884-887.
Mayordomo, J.I., Zorina, T., Storkus, W.J., Zitvogel, L., Celluzzi, C., Falo, L.D., Melief, C.J., Ildstad, S.T., Kast, W.M., and DeLeo, A.B. (1995). Bone marrow-derived dendritic cells pulsed with synthetic tumour peptides elicit protective and therapeutic antitumour immunity. Nat. Med. 1, 1297-1302.
Mitchison, N.A. (1955). Studies on the immunological response to foreign tumor transplants in the mouse. I. The role of lymph node cells in conferring immunity by adoptive transfer. J. Exp. Med. 102, 157-177.
Pope, C., Kim, S.K., Marzo, A., Masopust, D., Williams, K., Jiang, J., Shen, H., and Lefrancois, L. (2001). Organ-specific regulation of the CD8 T cell response to Listeria monocytogenes infection. J. Immunol. 166, 3402-3409.
Restifo, N.P., Dudley, M.E., and Rosenberg, S.A. (2012). Adoptive immunotherapy for cancer: harnessing the T cell response. Nat. Rev. Immunol. 12, 269-281.
Saito, T., and Batista, F.D. (2010). Immunological Synapse (Springer).
Schumacher, T.N., and Schreiber, R.D. (2015). Neoantigens in cancer immunotherapy. Science 348, 69-74.
Wolchok, J.D., and Chan, T.A. (2014). Cancer: Antitumour immunity gets a boost. Nature 515, 496-498.
Yadav, M., Jhunjhunwala, S., Phung, Q.T., Lupardus, P., Tanguay, J., Bumbaca, S., Franci, C., Cheung, T.K., Fritsche, J., Weinschenk, T., et al. (2014). Predicting immunogenic tumour mutations by combining mass spectrometry and exome sequencing. Nature 515, 572-576.

## Claims

1. Transinfected lymphocyte with a bacterium comprising a tumor antigen wherein the transinfected lymphocyte comprises the tumor antigen in the major histocompatibility complex I (MHC-I).

2. Lymphocyte according to claim 1 wherein the lymphocyte is CD4⁺ T cell and/or B cell

3. Lymphocyte according to any of claims 1 or 2 wherein the bacterium is selected from the list consisting of: *L. monocytogenes, Salmonella enterica, Mycobacterium bovis, Staphylococcus aureus* and *Escherichia coli.*

4. Lymphocyte according to any of claims 1 to 3 wherein the transinfection is performed from an antigen presenting cell.

5. Lymphocyte according to claim 4 wherein the antigen presenting cell is a dendritic cell.

6. Lymphocyte according to any of claims 1 to 5 wherein the lymphocyte is heterologous or autologous.

7. Lymphocyte according to any of claims 1 to 6 wherein the lymphocyte is from a human.

8. Cell population comprising the lymphocyte according to any of claims 1 to 7.

9. Pharmaceutical composition comprising the lymphocyte according to any of claims 1 to 7 or the cell population according to claim 8.

10. Use of the lymphocyte according to any of claims 1 to 7 or of the cell population according to claim 8 for the manufacture of a medicament.

11. Use of the lymphocyte according to any of claims 1 to 7 or of the cell population according to claim 8 for the manufacture of a medicament for the prevention or treatment of tumor and/or stimulating the immune response against a tumor antigen.

12. Use according claim 11 wherein the tumor is selected from the list consisting of: melanoma, lymphoma, chronic lymphocytic leukemia, myeloma, breast, ovary, uterus, cervix, testis, prostate, colon, colorectal, pancreatic, stomach and gastrointestinal tumors, gastric cancer, liver tumor, kidney, bladder, mouth cancer, pharynx, larynx, esophagus, lung, thyroid, glioblastoma, glioma, sarcoma, encephalon, brain, neuroblastoma and marrow, head and neck blastoma, bone and connective tissue.

13. Kit or device comprising the lymphocyte according to any of claims 1 to 7 or the cell population according to claim 8.

14. Use of the kit or device according to claim 13 for prevention or treatment of tumor and/or stimulating the immune response against a tumor antigen.

15. Use according to claim 14 wherein the tumor is selected from the list consisting of: melanoma, lymphoma, chronic lymphocytic leukemia, myeloma, breast, ovary, uterus, cervix, testis, prostate, colon, colorectal, pancreatic, stomach and gastrointestinal tumors, gastric cancer, liver tumor, kidney, bladder, mouth cancer, pharynx, larynx, esophagus, lung, thyroid, glioblastoma, glioma, sarcoma, encephalon, brain, neuroblastoma and marrow, head and neck blastoma, bone and connective tissue.

16. *In vitro* method for transinfecting a lymphocyte comprising the following steps:
a. Isolating an antigen-presenting cell and a lymphocyte from a biological sample;
b. differentiating the antigen-presenting cell;
c. infecting the antigen presenting cell of step (b) with a bacterium, where the bacterium comprises a tumor antigen;
d. contacting the antigen-presenting cell infected in step (c) with the lymphocyte of step (a) at a temperature of 35-38 ° C for 24-72 hours to transinfecting it.

17. Method of claim 16 wherein it further comprises a step (e) for the selection of the transinfected lymphocyte of step (d).

18. Method of claim 16 or 17 wherein the bacterium is selected from the list consisting of: *L. monocytogenes, Salmonella enterica, Mycobacterium bovis, Staphylococcus aureus* and *Escherichia coli.*

19. Method according to any of claims 16 to 18 wherein the antigen presenting cell is a dendritic cell.

20. Method according to any of claims 16 to 19 wherein the lymphocyte is CD4⁺ T cell and/or B cell.

21. Method according to any of claims 16 to 20 wherein the lymphocyte is autologous or heterologous.

22. Method according to any of claims 16 to 21 wherein in step (d) the temperature is 37° C for 48 hours.


SEQUENCE LISTING

<110>  Consejo Superior de Investigaciones Científicas
       Universidad Autónoma de Madrid
       Fundación de Investigación Biomédica del Hospital
       Universitario la Princesa

<120>  Linfocitos transinfectados para terapia anti-tumoral

<130>  PCT1641.1067

<150>  ES P201531177
<151>  2015-08-07

<160>  3

<170>  PatentIn version 3.5

<210>  1
<211>  8
<212>  PRT
<213>  Artificial Sequence

<220>
<223>  péptido OVAp-I 257-264

<400>  1

Ser Ile Ile Asn Phe Glu Lys Leu
1               5


<210>  2
<211>  16
<212>  PRT
<213>  Artificial Sequence

<220>
<223>  péptido MCCp 88-103

<400>  2

Ala Asn Glu Arg Ala Asp Leu Ile Ala Tyr Leu Lys Gln Ala Thr Lys
1               5                   10                  15


<210>  3
<211>  17
<212>  PRT
<213>  Artificial Sequence

<220>
<223>  péptido OVA 323-339

<400>  3

Ile Ser Gln Ala Val His Ala Ala His Ala Glu Ile Asn Glu Ala Gly
1               5                   10                  15


Arg